(19)

Europäisches Patentamt
European Patent Office
Office européen des brevets

(11) **EP 3 926 035 A1**

(12) **EUROPEAN PATENT APPLICATION**
published in accordance with Art. 153(4) EPC

(43) Date of publication:
**22.12.2021  Bulletin 2021/51**

(21) Application number: **20755593.9**

(22) Date of filing: **14.01.2020**

(51) Int Cl.:
*C12M 1/36* (2006.01)          *C12M 1/34* (2006.01)
*G05B 13/04* (2006.01)

(86) International application number:
**PCT/KR2020/000656**

(87) International publication number:
**WO 2020/166831 (20.08.2020 Gazette 2020/34)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **15.02.2019   KR 20190017574**

(71) Applicants:
  • **CJ Cheiljedang Corporation
    Seoul 04560 (KR)**
  • **Seoul National University R & DB Foundation
    Seoul 08826 (KR)**

(72) Inventors:
  • **KIM, Dae Shik
    Seongnam-si, Gyeonggi-do 13465 (KR)**

  • **BANG, Seong Eun
    Yongin-si, Gyeonggi-do 16948 (KR)**
  • **SHIN, Jong Hwan
    Gimpo-si, Gyeonggi-do 10073 (KR)**
  • **KIM, Il Chul
    Seoul 06217 (KR)**
  • **PARK, Seon Mi
    Seoul 06997 (KR)**
  • **LEE, Jong Min
    Seoul 06548 (KR)**
  • **BAE, Jae Han
    Seoul 08785 (KR)**
  • **LEE, Hye Ji
    Seoul 07324 (KR)**
  • **KIM, Jung Hun
    Seoul 08788 (KR)**

(74) Representative: **Bandpay & Greuter
30, rue Notre-Dame des Victoires
75002 Paris (FR)**

(54)  **DEVICE AND METHOD FOR DETERMINING OPERATION CONDITION OF BIOREACTOR**

(57)    Disclosed are a method and a device for determining an operating condition of a bioreactor. Each of the method and the device receives N experimental data sets, models the bioreactor using the N experimental data sets to construct a bioreactor model, and determines an optimal operation condition based on an operation scheme of the bioreactor, using the constructed bioreactor model.

```
                    START
                      │
                      ▼
┌──────────────────────────────────────────┐
│      RECEIVE N EXPERIMENTAL DATA SETS     │──S10
└──────────────────────────────────────────┘
                      │
                      ▼
┌──────────────────────────────────────────┐
│   CONSTRUCT BIOREACTOR MODEL USING        │──S20
│        EXPERIMENTAL DATA SETS             │
└──────────────────────────────────────────┘
                      │
                      ▼
┌──────────────────────────────────────────┐
│  DETERMINE OPTIMAL OPERATION CONDITION OF │──S30
│ BIOREACTOR USING CONSTRUCTED BIOREACTOR MODEL│
└──────────────────────────────────────────┘
                      │
                      ▼
┌──────────────────────────────────────────┐
│   OUTPUT DETERMINED OPTIMAL OPERATION     │──S40
│              CONDITION                     │
└──────────────────────────────────────────┘
                      │
                      ▼
                    END
```

FIG.3

EP 3 926 035 A1

**Description**

[Technical Field]

The present disclosure relates to a device and a method for determining an operation condition of a bioreactor.

[Background Art]

The importance of methods for producing energy fuels, nutrients, petroleum-derived organic compounds, etc. from microbial (bacteria-based) fermentation processes is increasing worldwide. For example, L-amino acids are basic building blocks of proteins. Various methods for industrially producing L-amino acids from microorganisms are being studied. Various studies are being conducted to develop microorganisms that produce products such as L-amino acids at high efficiency (Korean Patent No. 10-0924065 and Korean patent No. 1208480). However, there is a lack of research on the microbial fermentation process on an industrial scale, particularly a process for obtaining consistent fermentation results.

This commercial microbial fermentation process or bioreaction process is being carried out depending on experiences of a producer and past operation results. In this case, it is not possible to determine whether a current operation strategy is optimal or not, so that maximum performance of a strain used in the current process cannot be derived.

Further, when the operation strategy is changed, an effect of the change on a production amount cannot be predicted. Thus, it is difficult to quickly apply change in a production target according to market change to an actual process.

Conventionally, behavior itself of the strain has been mathematically modeled. A result of modeling only a specific strain accurately simulates the behavior of the strain, but is inevitably low in a predictive power for a strain whose performance is improved via genetic improvement.

Under this background, the present inventors have performed intensive research to establish an optimal operation condition to obtain a target result in the fermentation process of microorganisms, and thus have constructed a bioreactor model and have derived an optimal operation condition. In this way, the present disclosure has been completed.

[Disclosure]

[Technical Problem]

The present disclosure intends to provide a bioreactor operation condition determination device and a bioreactor operation condition determination method for modeling a bioreactor based on actual process data and deriving an optimal operation condition of the bioreactor using a thus-constructed bioreactor model.

[Technical Solution]

In one embodiment, a method for determining an operating condition of a bioreactor includes receiving N experimental data sets, modeling the bioreactor using the N experimental data sets to construct a bioreactor model, and determining an optimal operation condition based on an operation scheme of the bioreactor, using the constructed bioreactor model.

The experimental data set may include a state variable and an operation condition of a previously-performed process.

The constructing of the bioreactor model may include defining one experimental data set among the N experimental data sets as a validation data set, and defining remaining N-1 experimental data sets or some thereof as a training data set, estimating a parameter set using the training data set, creating candidate bioreactor models using the estimated parameter set, validating simulation performance of each of the created candidate bioreactor models, using the validation data set, and selecting a final bioreactor model among the created candidate bioreactor models, based on the simulation performance validation result.

The validating of the simulation performance of each of the candidate bioreactor models may include comparing a predicted value of each of the candidate bioreactor models with a measurement value of the validation data set, and calculating a validation error based on the comparing result.

The selecting of the final bioreactor model may include selecting a candidate bioreactor model having a smallest validation error as the final bioreactor model, from among the candidate bioreactor models.

The validating of the simulation performance of each of the candidate bioreactor models may include iteratively performing the validating of the simulation performance of each of the candidate bioreactor models while changing the training data set and the validation data set constituting the N experimental data sets.

The determining of the optimal operation condition may include dividing an entire operation time duration of the bioreactor into a plurality of time-elements, calculating change in a state variable based on each time-element-based substrate feed rate, using the constructed bioreactor model, and determining an optimal substrate feed rate that minimizes an objective function according to the operation scheme of the bioreactor, based on a calculation result of the change in the state variable.

The dividing of the entire operation time duration of the bioreactor into the plurality of time-elements may include selecting a substrate feed rate change frequency, calculating a substrate feed start time, dividing a time duration from the substrate feed start time to an end time of an operation of the bioreactor into the plurality of time-elements, based on the substrate feed rate change frequency, and calculating a start time point of each of the time-elements.

The objective function may be set to one of a yield and a productivity.

The operation scheme may include a CBO (Continuous Broth Output) scheme or a MBO (Middle Broth Output) scheme.

The determining of the optimal feed rate may include calculating a time-point at which a reactor volume reaches a maximum volume when the operation scheme is the MBO scheme, setting the reactor volume at the calculated time-point to a value obtained by subtracting a MBO volume from the reactor volume, wherein the MBO volume is a product volume discharged from the bioreactor, storing a result of calculating change in the state variable for a duration from a start time-point of a time-element in which the reactor volume reaches the maximum volume to the calculated time-point, and calculating change in the state variable for a duration from the calculated time-point to an end time-point of the time-element in which the reactor volume reached the maximum volume, using the constructed bioreactor model, and storing the change.

In another embodiment, a device for determining an operation condition of a bioreactor includes a memory, and a processor connected to the memory, wherein the processor receives N experimental data sets, models the bioreactor using the N experimental data sets to construct a bioreactor model, and determines an optimal operation condition based on an operation scheme of the bioreactor, using the constructed bioreactor model.

The experimental data set may include a state variable and an operation condition of a previously-performed process, wherein the state variable may include at least one of a cell concentration, a substrate concentration, a reactor volume, or a product concentration, and wherein the operation condition may include at least one of a substrate feed rate, a temperature, pH (hydrogen exponent) or the operation scheme.

The processor may define one experimental data set among the N experimental data sets as a validation data set, and define remaining N-1 experimental data sets or some thereof as a training data set, estimate a parameter set using the training data set, create candidate bioreactor models using the estimated parameter set, validate simulation performance of each of the created candidate bioreactor models, using the validation data set, and select a final bioreactor model among the created candidate bioreactor models, based on the simulation performance validation result.

The processor may compare a predicted value of each of the candidate bioreactor models with a measurement value of the validation data set, and calculate a validation error based on the comparing result.

The processor may select a candidate bioreactor model having a smallest validation error as the final bioreactor model, from among the candidate bioreactor models.

The processor may iteratively perform the validating of the simulation performance of each of the candidate bioreactor models while changing the training data set and the validation data set constituting the N experimental data sets.

The processor may divide an entire operation time duration of the bioreactor into a plurality of time-elements, calculate change in a state variable based on each time-element-based substrate feed rate, using the constructed bioreactor model, and determine an optimal substrate feed rate that minimizes an objective function according to the operation scheme of the bioreactor, based on a calculation result of the change in the state variable.

The processor may select a substrate feed rate change frequency, calculate a substrate feed start time, divide a time duration from the substrate feed start time to an end time of an operation of the bioreactor into the plurality of time-elements, based on the substrate feed rate change frequency, and calculate a start time point of each of the time-elements.

The processor may calculate a time-point at which a reactor volume reaches a maximum volume when the operation scheme is a MBO scheme, set the reactor volume at the calculated time-point to a value obtained by subtracting a MBO volume from the reactor volume, wherein the MBO volume is a product volume discharged from the bioreactor, store a result of calculating change in the state variable for a duration from a start time-point of a time-element in which the reactor volume reaches the maximum volume to the calculated time-point, calculate change in the state variable for a duration from the calculated time-point to an end time-point of the time-element in which the reactor volume reached the maximum volume, using the constructed bioreactor model, and store the change.

[Advantageous Effects]

According to embodiments of the present disclosure, the bioreactor may be modeled based on the actual process data and then the behavior of the bioreactor may be predicted using the thus-constructed bioreactor model, thereby deriving the optimal operation condition of the bioreactor.

Further, according to embodiments of the present disclosure, the modeling may be performed based on the actual process data. Thus, the modeling may be applied to a bioreactor using a genetically modified strain. The modeling may be performed directly using the actual process data, such that there is no need to worry about scale-up.

Further, according to embodiments of the present disclosure, the method may be performed so as to minimize the objective function specified by a user based on the actual process data. Thus, an operation strategy may be derived which may follow an operation objective varying depending on a situation of an amino acid market, and may maximize performance of the strain being used at a time-point of interest.

[Description of Drawings]

FIG. 1 is a configuration diagram showing a device for determining an operation condition of a bioreactor according to an embodiment of the present disclosure.

FIG. 2 is a graph showing behavior of a process indicator predicted by actual process data and a bioreactor model related to the present disclosure.

FIG. 3 is a flowchart illustrating a method for determining a bioreactor operation condition according to an embodiment of the present disclosure.

FIG. 4 is a flow chart showing a process of constructing a bioreactor model shown in FIG. 3.

FIG. 5 is a flow chart showing a process of determining an operation condition using the bioreactor model shown in FIG. 3.

FIG. 6 is a graph showing change in a process indicator when operating the bioreactor according to the present disclosure.

[Mode for Invention]

Hereinafter, some embodiments of the present disclosure will be described in detail with reference to the exemplary drawings. In adding the reference numerals to the components of each drawing, it should be noted that the identical or equivalent component is designated by the identical numeral even when they are displayed on other drawings. Further, in describing the embodiment of the present disclosure, a detailed description of the related known configuration or function will be omitted when it is determined that it interferes with the understanding of the embodiment of the present disclosure.

In describing the components of the embodiment according to the present disclosure, terms such as first, second, A, B, (a), (b), and the like may be used. These terms are merely intended to distinguish the components from other components, and the terms do not limit the nature, order or sequence of the components. Unless otherwise defined, all terms including technical and scientific terms used herein have the same meaning as commonly understood by one of ordinary skill in the art to which this disclosure belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

The present disclosure relates to a technique for modeling a bioreactor based on actual process data using the bioreactor and deriving an optimal operation condition of the bioreactor using a thus-constructed model.

For example, according to embodiments of the present disclosure, a bioreactor used to produce lysine may be modeled based on actual process data including a state variable and an operation condition (operation strategy) of a previously-performed process, and based on behavior characteristics of the genus Corynebacterium strain. Then, various operation strategies may be simulated with a dynamic optimization technique using a thus-constructed bioreactor model. Thus, an algorithm to determine an optimal operation strategy that minimizes an objective function set by the user may be derived. In this way, a process performance indicator such as productivity or yield may be maximized according to a purpose of interest.

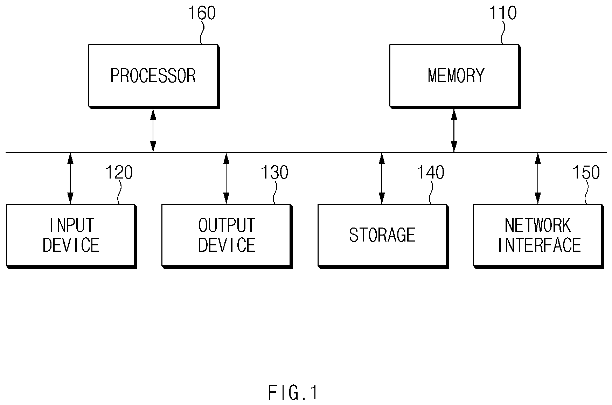

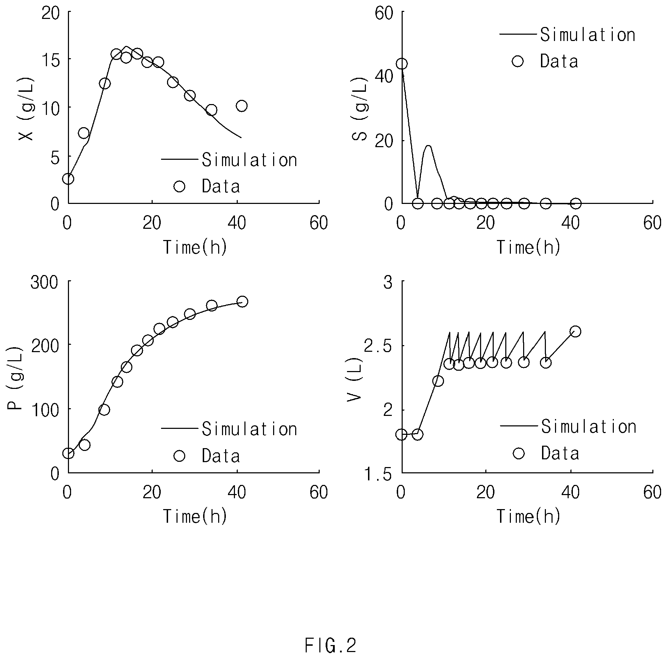

FIG. 1 is a configuration diagram showing a device for determining an operation condition of a bioreactor according to an embodiment of the present disclosure. FIG. 2 is a graph showing behavior of a process indicator predicted by actual process data and a bioreactor model related to the present disclosure. The device for determining the operation condition of the bioreactor according to the present disclosure may be implemented using a computing device.

Referring to FIG. 1, the device for determining the operation condition of the bioreactor includes a memory 110, an input device 120, an output device 130, storage 140, a network interface 150, and a processor 160.

The memory 110 may store therein a program for operation of the processor 160, and may temporarily store therein input data and output data.

Further, the memory 110 may store therein an algorithm for constructing a model of the bioreactor and an algorithm for optimizing an optimal operation strategy of the bioreactor. The memory 110 may store therein actual process data including a state variable and an operation condition of a previously-performed process, that is, an experimental data set.

The memory 110 may be implemented as at least one storage medium (recording medium) among storage media such as a flash memory, a hard disk, a SD card (Secure Digital Card), RAM (Random Access Memory), ROM (Read Only Memory), EEPROM (Electrically Erasable and Programmable ROM), EPROM (Erasable and Programmable ROM), a register, and a removable disk.

The input device 120 is a device for inputting data. The input device 120 may be implemented as at least one among input devices such as a keypad, a keyboard, a dome switch, a touch pad, and a touch screen. Further, the input device 120 may be implemented as a barcode reader and a magnetic ink character reader.

The output device 130 outputs various information or data in a form of visual information and/or auditory information under an operation of the processor 160. The output device 130 may include a display device such as a liquid crystal display (LCD), a thin film transistor-liquid crystal display (TFT LCD), an organic light-emitting diode (OLED) display, a flexible display, a three-dimensional display (3D display) and/or a transparent display, and/or an audio output device such as a receiver, a speaker and/or a buzzer.

The storage 140 may include various types of storage media as in the memory 110. The storage 140 may be implemented as a web storage that performs a storage function of the memory 110 on the Internet. The storage 140 may store therein experimental data.

The network interface 150 enables wired/wireless communication with other terminals via a network. The network interface 150 may use at least one of communication technologies such as wireless Internet, mobile communication, and short-range communication. The wireless Internet technology may include WLAN (Wireless LAN: WiFi), Wibro (Wireless broadband), and/or Wimax (World Interoperability for Microwave Access). The short-range communication technology may include Bluetooth, NFC (Near Field Communication), RFID (Radio Frequency Identification), infrared communication (IrDA: infrared Data Association), UWB (Ultra Wideband), and/or ZigBee. The mobile communication technology may include CDMA (Code Division Multiple Access), GSM (Global System for Mobile communication), LTE (Long Term Evolution) and/or LTE-Advanced.

The processor 160 executes a program and/or an algorithm stored in the memory 110. The processor 160 may be implemented using at least one of an ASIC (Application Specific Integrated Circuit), DSP (Digital Signal Processor), PLD (Programmable Logic Device), FPGA (Field Programmable Gate Array), CPU (Central Processing unit), a microcontroller, or a microprocessor.

The processor 160 creates (constructs) a bioreactor model via mathematical modeling of the bioreactor using the actual process data of the bioreactor. The processor 160 determines an optimal operation condition or optimal operation strategy of the bioreactor to obtain process performance desired by the user using the constructed bioreactor model.

Hereinafter, a process of constructing the bioreactor model will be described in detail. The process of constructing the bioreactor model may largely include a process of defining the bioreactor using Equations and a process of estimating a parameter set included in the Equations.

The bioreactor may be represented by mole balance equations such as following Equation 1 to Equation 4.

Equation 1

$$\frac{dX}{dt} = \mu X - DX$$

Equation 2

$$\frac{dS}{dt} = -\left(\rho + \frac{\mu}{Y_{X/S}} + \frac{\pi}{Y_{P/S}}\right) X + D(S_{in} - S)$$

Equation 3

$$\frac{dP}{dt} = \pi X - DP$$

## Equation 4

$$\frac{dV}{dt} = DV$$

$\mu$ and n included in the above-described mole balance equations may be expressed using following Equation and Equation 6.

## Equation 5

$$\mu = \mu_m \frac{S}{K_s + S} - \mu_d$$

## Equation 6

$$\pi = \alpha\mu + \beta$$

A dilution rate D due to input of a substrate may be expressed using following Equation 7 and Equation 8 according to an operation scheme of the bioreactor. The operation scheme of the bioreactor may be classified into CBO (Continuous Broth Out) which continuously outputs a product, and MBO (Middle Broth Out) which outputs a product in a middle of an operation. When the operation scheme of the bioreactor is MBO, the dilution rate D may be expressed using a following Equation 7.

## Equation 7

$$D = \frac{u_{in}}{V}$$

When the operation scheme of the bioreactor is CBO, the dilution rate D may be expressed using a following Equation 8.

## Equation 8

$$D = \frac{u_{in} - u_{out}}{V}$$

The state variables (a performance indicator, and a process indicator) X, S, V and P included in the above Equation 1 to Equation 8 are defined as in Table 1.

Table 1

| State variable | Unit | Meaning |
|---|---|---|
| X | $gL^{-1}$ | Cell concentration |
| S | $gL^{-1}$ | Sugar or substrate concentration |
| V | L | Effective volume of reactor |
| P | $gL^{-1}$ | Product concentration |

The parameters (parameter set) included in the above Equation 1 to Equation 8 are defined as in Table 2.

Table 2

| Parameter | Unit | Meaning |
|---|---|---|
| $S_{in}$ | $gL^{-1}$ | Concentration of substrate fed to reactor |
| $\mu_m$ | $h^{-1}$ | Maximum biomass growth rate |

(continued)

| Parameter | Unit | Meaning |
|---|---|---|
| $\mu_d$ | $h^{-1}$ | Maximum biomass death rate |
| $K_s$ | $gL^{-1}$ | Half-coefficient for cellular growth (substrate effect) |
| $\alpha$ | - | Growth-dependent production rate |
| $\beta$ | $h^{-1}$ | Growth-independent production rate |
| P | $h^{-1}$ | Substrate consumption rate |
| $Y_{x/s}$ | - | Yield coefficient between X and S |
| $Y_{p/s}$ | - | Yield coefficient between P and S |
| $u_{in}$ | - | Substrate feed rate |
| $u_{out}$ | - | Substrate output rate |

As described above, the bioreactor operating in the MBO scheme may be represented by Equation 1 to Equation 7, while the bioreactor operating in the CBO scheme may be represented by Equation 1 to Equation 6 and Equation 8.

Thereafter, the parameter set included in the equations corresponding to the bioreactor operating in each operation scheme MBO or CBO is estimated. That is, in order to construct a bioreactor model operating in the MBO scheme, the parameter set included in Equation 1 to Equation 7 is estimated. On the other hand, in order to construct a bioreactor model operating in the CBO scheme, the parameter set included in Equation 1 to Equation 6 and Equation 8 is estimated.

A process of estimating the parameter set for construction of the bioreactor model operating in the MBO scheme is the same as a process of estimating the parameter set for construction of the bioreactor model operating in the CBO scheme. In the present disclosure, a parameter set estimation process for constructing a bioreactor model operating in the MBO scheme will be described as a representative example.

First, the processor 160 receives at least two experimental data sets. In this connection, each experimental data set may refer to data used in the actual process, and may include a state variable and an operation condition of a previously-performed process using the bioreactor. The state variable may include at least one of a cell concentration, a substrate concentration, a reactor volume, or a product concentration. The operation condition may mean an input value required to operate the process and may include at least one of a substrate feed rate, a temperature, pH (hydrogen exponent) or an operation scheme (CBO or MBO). The processor 160 may receive an experimental data set input through the input device 120 or the network interface 150. Further, the processor 160 may access the experimental data set stored in the memory 110 or the storage 140 as input data.

The processor 160 may define one experimental data set of N experimental data sets as a validation data set and may define the remaining N-1 experimental data sets or some thereof as a training data set.

The processor 160 may estimate the parameter set according to the operation scheme using the training data set, and complete the bioreactor model based on characteristics of the cell using the estimation result. In other words, when the operation scheme is MBO, the processor 160 any estimate the parameter set included in Equation 1 to Equation 7 using the training data set, and apply the estimated parameter set into Equation 1 to Equation 7, thereby completing the bioreactor model operating in the MBO scheme. On the other hand, when the operation scheme is CBO, the processor 160 may estimate the parameter set included in Equation 1 to Equation 6 and Equation 8 using the training data set, and may apply the estimated parameter set into Equation 1 to Equation 6 and Equation 8, thereby completing the bioreactor model operating in the CBO scheme.

More specifically, the processor 160 estimates values of the parameter set $P_i = \{p_1, p_2,..., p_8\}$ using the training data set. In this connection, the parameter set $P_i$ includes values of parameters $p_i$ listed in Table 2. The processor 160 estimates the parameter set $P_i$ that best simulates N-1 experimental data sets included in the training data set or some thereof, using a genetic algorithm (GA) as a global optimization algorithm.

The processor 160 validates simulation performance of the bioreactor model created based on the estimated parameter set Pi, using the validation data set (that is, validation). The processor 160 creates a candidate bioreactor model by applying the parameter values included in the estimated parameter set to the above Equation 1 to Equation 7 or the above Equation 1 to Equation 6 and Equation 8 depending on the operation scheme. In this connection, the candidate bioreactor model may be defined as a bioreactor model created using each estimated parameter set Pi.

The processor 160 validates the simulation performance of the created candidate bioreactor model using the validation data set. That is, the processor 160 compares a value measured experimentally at a sampling point (that is, a measured value of the validation data set) with a value predicted using the candidate bioreactor model (that is, a predicted value of the candidate bioreactor model) and then calculates a sum of normalized square errors, that is, an validation error, and then store the calculation result. In this connection, the sampling point may be defined as a time-point at which a state variable value is measured in a bioreactor production process. For example, referring to FIG. 2, the processor 160 compares a cell concentration X, a substrate concentration S, a reactor volume V, and a product concentration P as experimentally measured with a cell concentration X, a substrate concentration S, a reactor volume V, and a product concentration P predicted using the bioreactor model, respectively, and then checks simulation performance of the bioreactor model based on the comparing result. In other words, the processor 160 determines the simulation performance of the bioreactor model based on similarity between the experimentally measured state variable value and the state variable value predicted by the bioreactor model.

The processor 160 determines the training data set and the validation data set different from each other from among the N experimental data sets and then repeatedly performs the calculation of the validation error using the validation data set. Thus, performance for each of multiple combinations of bioreactor models may be validated. Specifically, the processor 160 performs bioreactor modeling and cross-validation based on all combinations of validation data sets and training data sets derived from the N experimental data sets. For example, when experimental data sets are D1, D2, and D3, D1 is defined as the validation data set, while D2 and D3 are defined as the training data set. Then, the bioreactor modeling is performed using D2 and D3, and then the simulation performance of the bioreactor model is validated using D1. Next, D2 is defined as the validation data set, while D1 and D3 are defined as the training data set. Then, the bioreactor modeling is performed using D1 and D3, and then simulation performance validation of the bioreactor model is performed using D2. Finally, D3 is defined as the validation data set, while D1 and D2 are defined as the training data set. Then, the bioreactor modeling is performed using D1 and D2, and then simulation performance validation of the bioreactor model is performed using D3.

The processor 160 selects, as a final bioreactor model, a candidate bioreactor model having the best simulation performance based on the validation data set among candidate bioreactor models. The processor 160 uses a validation error as an objective function to select a parameter set having the smallest validation error among the estimated parameter sets. In the present disclosure, the objective function may be defined as a function used for a certain purpose. In the process of constructing the bioreactor model, the objective function is configured to select a parameter set that minimizes the validation error. In the process of determining the optimal operation condition which will be described later, the objective function is configured to find the optimal operation condition that maximizes yield and productivity.

Next, a method for determining the optimal operation condition of the bioreactor using the constructed bioreactor model (that is, the selected final bioreactor model) will be described in detail. In this connection, the optimal operation condition may be an optimal feed rate of a substrate in a time-element. For example, an entire operation time duration of the bioreactor is divided into multiple time-elements. Then, change in the state variable (e.g., cell concentration, substrate concentration, reactor volume and product concentration) is calculated using the constructed bioreactor model and based on a feed rate optimal of a substrate in a time-element. Based on a result of calculating the change in the state variable, an optimal substrate feed rate (hereinafter, optimal feed rate) that minimizes the objective function in terms of the change in the state variable calculated according to the operation scheme of the bioreactor is calculated.

First, the processor 160 selects a substrate feed rate change frequency K and an objective function J according to a user input. In this connection, the substrate feed rate change frequency K may mean the number of times to change a feed rate of the substrate for the entire operation time duration of the bioreactor, and may be arbitrarily designated. The objective function J may be selected relative to yield or productivity.

The processor 160 divides the entire operation time duration of the bioreactor into K+1 time-elements based on the selected substrate feed rate change frequency K, and calculates change in the state variable based on each time-element-based substrate feed rate and using the constructed bioreactor model. In this connection, a minimum and a maximum of the time-element-based substrate feed rate may employ previously-performed process data (that is, actual process data) as empirically obtained.

More specifically, the processor 160 calculates a time-point tfs at which the substrate in the bioreactor is completely consumed in a first time-element for which the substrate is not fed thereto. The processor 160 calculates a time-point $t_k$ (k=1,..., K+1) corresponding to a start time point of each of K+1 time-elements into which a time duration from the time-point tfs to an end time-point $t_f$ of the bioreactor operation is divided into.

The processor 160 parameterizes a substrate feed rate in each of the K+1 time-elements, that is, inputs a substrate feed rate as a control input to the constructed bioreactor model. In this connection, the processor 160 parameterizes the time-element-based optimal substrate feed rate using CVP (Control Vector Parameterization). The processor 160 calculates the change in the state variable by applying the substrate feed rate as a parameter to the bioreactor model. When the substrate feed rate as the parameter is applied to the bioreactor model, the model may be expressed using an ordinary differential equation (ODE).

The processor 160 calculates the optimal feed rate in a condition varying according to the operation scheme of the bioreactor. First, when the operation scheme is MBO, the processor 160 operates the bioreactor as a fed-batch bioreactor. When a volume V of the bioreactor reaches a maximum reactor volume $V_{max}$, the processor 160 calculate the optimal feed rate in a condition to allow MBO having VMBO = 0.25L to occur. In this connection, VMBO refers to a volume of a product outputted from the bioreactor, and may be arbitrarily set by an operator. $V_{max}$ may be set to a value varying depending on the bioreactor.

The processor 160 derives, as the optimal operation condition, an operation condition of the bioreactor that maximizes the objective function J based on the calculated change in the state variable. That is, the processor 160 determines the optimal feed rate of the bioreactor that maximizes yield or productivity.

The objective function J is defined by multiplying the yield or productivity by a negative number. The yield may refer to a ratio of an entire production amount relative to a substrate input amount during the entire operation time duration of the bioreactor, and may be expressed using Equation 9. In this connection, the entire production amount is a sum of an amount of a product in the bioreactor at the end time-point of the operation of the bioreactor and a product amount in a solution extracted in MBO or CBO during the operation thereof.

### Equation 9

$$\text{Yield} = \frac{Total\ product\ mass\ (at\ final\ time, including\ MBOs)}{Total\ supplied\ Substrate\ mass}$$

The productivity may refer to an amount (ton/KL year) that may be produced for one year including a batch cleaning time, and may be expressed using Equation 10.

### Equation 10

$$\text{Productivity} = \frac{Total\ product\ mass}{t_{final}+8} \times 24 \times \frac{365}{4.0} \times \frac{0.97}{1000}$$

In this connection, $t_{final}$ means an end time of the operation of the bioreactor.

In order to maximize the yield, as in Equation 11, the objective function $J_{Yield}$ is defined by multiplying the yield by a negative number. The objective function $J_{Yield}$ includes a Productivity-Productivityheuristic term that indicates a difference between a productivity of the previous operation and that of a current operation such that the current operation exhibits similar productivity to that of the previous operation when the current operation uses a new substrate. Further, the objective function $J_{Yield}$ includes an input change term to ensure that the change in the time-element-based feed rate does not become excessively large because of the realistic limitations of the bioreactor.

### Equation 11

$$J_{Yield} = -A \ast Yield + B \ast (Input\ change)^2 + C \ast (Productivity - Productivity_{heuristic})^2$$

In order to maximize the productivity, as in Equation 12, the objective function $J_{Productivity}$ includes a value obtained by multiplying the productivity by a negative number. The objective function $J_{Productivity}$ includes a $S_{consumption}$ term that indicates a consumption of the substrate so that a process that inefficiently increases productivity using an excessive amount of the substrate is not determined as an optimal process. Further, the objective function $J_{Productivity}$ includes an input change term that indicates the change in the time-element-based feed rate based on the limitations of the bioreactor.

### Equation 12

$$J_{Productivity} = -A \ast Productivity + B \ast (Input\ change)^2 + C \ast S_{consumption}$$

In each of the objective function $J_{Yield}$ and $J_{Productivity}$, each of A, B, and C may denote a proportional constant that relatively expresses a weight of each term, that is, a scaling factor, and may be positive. Each of the scaling factors A, B, and C may serve to scale an order of magnitude of each term so that the order of magnitude of each term does not exhibit a large difference. In this connection, each of the scaling factors A, B and C may be arbitrarily adjusted by the user. The processor 160 uses the genetic algorithm GA to calculate a substrate feed rate that minimizes the objective functions $J_{Yield}$ and $J_{Productivity}$.

In another example, when the operation scheme is CBO, the processor 160 calculates the optimal feed rate at a continuous stirred tank reactor (CSTR) condition in which an input flow rate and an output flow rate are set to be equal to each other and thus a volume is constant. When the operation scheme is CBO, the processor 160 may divide the entire operation time duration of the bioreactor into K+1 time-elements, and calculates the time-element-based optimal feed rate.

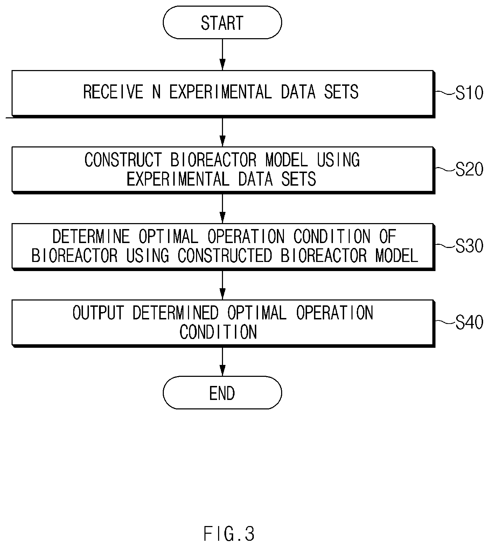

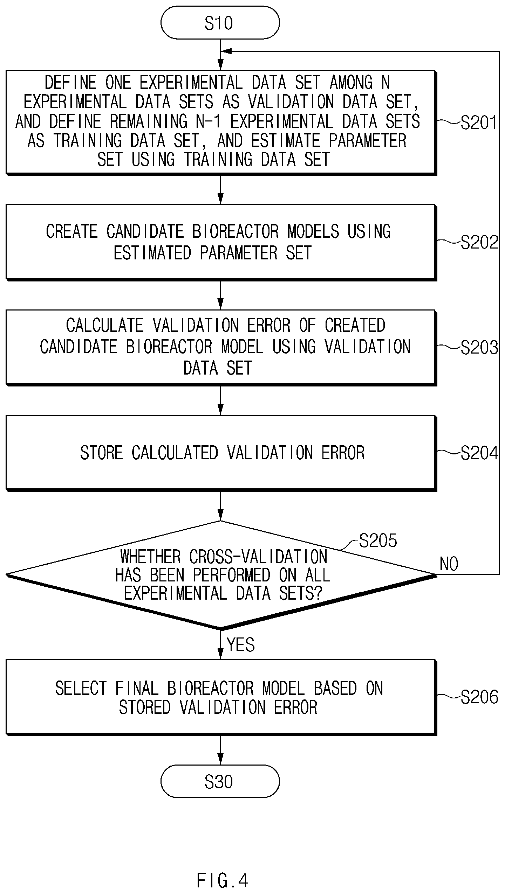

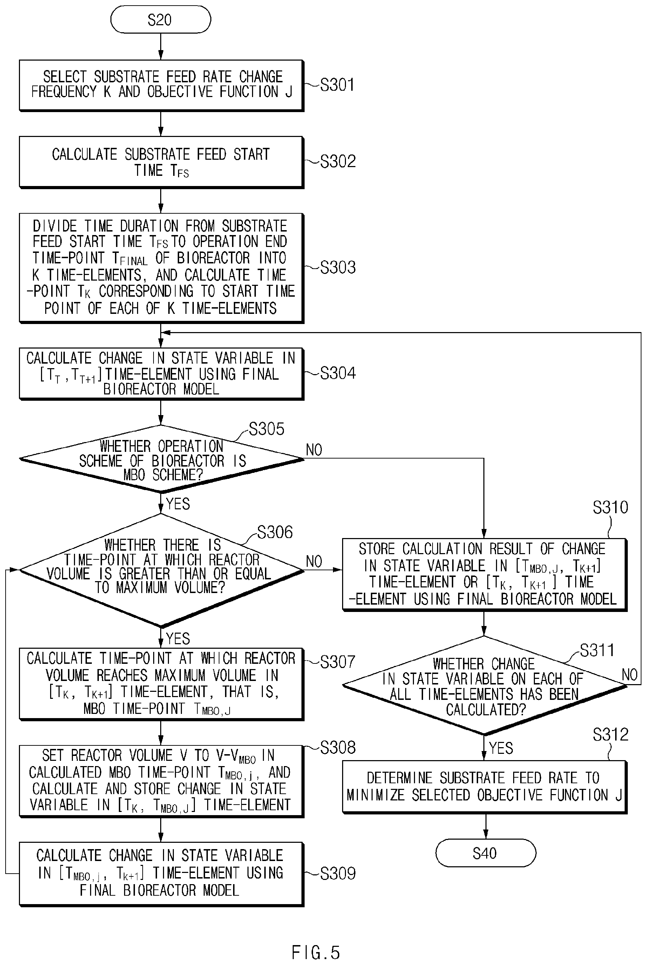

FIG. 3 is a flowchart illustrating a method for determining a bioreactor operation condition according to an embodiment of the present disclosure. FIG. 4 is a flowchart illustrating a process of constructing the bioreactor model shown in FIG. 3. FIG. 5 is a flowchart illustrating a process of determining an optimal operation condition using the model shown in FIG. 3.

The processor 160 receives the N experimental data sets S10. In this connection, the experimental data set refers to a data set obtained under a test using the same strain, and includes an operation condition and a state variable value (measurement value) of a previously-performed process in the bioreactor.

The processor 160 models the bioreactor based on the experimental data set to construct or create the bioreactor model S20. The process of constructing the bioreactor model is divided into a process of representing the bioreactor using the equations and a process of estimating the parameter set included in the equations. In S20, it is assumed that the process of representing the bioreactor using the equations has been completed.

More specifically, when describing the process S20 of constructing the bioreactor model with reference to FIG. 4, the processor 160 defines one experimental data set among the N experimental data sets as a validation data set, and defines the remaining N-1 experimental data sets or some thereof as the training data set, and then estimates the parameter set using the remaining N-1 experimental data sets or some thereof as the training data set S201. In this connection, the processor 160 estimates the parameter set using the genetic algorithm (GA). The processor 160 estimates the parameter set included in Equation 1 to Equation 7 based on the training data set when the operation scheme of the bioreactor is MBO. When the operation scheme is CBO, the parameter sets included in Equation 1 to Equation 6 and Equation 8 are estimated using the training data set.

The processor 160 creates candidate bioreactor models by applying the estimated parameter set into the equations corresponding to the operation scheme of the bioreactor S202. That is, the processor 160 may apply the estimated parameter set into Equation 1 to Equation 7 to complete the candidate bioreactor models operating in the MBO scheme. In another example, the processor 160 may apply the estimated parameter set in Equation 1 to Equation 6 and Equation 8 to complete the candidate bioreactor models operating in the CBO scheme.

The processor 160 calculates the validation error of the created candidate bioreactor model using the validation data set S203. In other words, the processor 160 validates the simulation performance of the candidate bioreactor model based on the validation data set.

The processor 160 stores the calculated validation error S204. That is, the processor 160 stores the validation result of the candidate bioreactor model, (that is, the simulation performance validation result).

The processor 160 identifies whether the cross-validation has been performed on all combinations of the validation data set and the training data sets that may constitute the N experimental data sets S205. The processor 160 repeatedly performs S201 to S204 until the cross validation on all experimental data sets is completed.

The processor 160 selects the final bioreactor model based on the stored validation error S206. The processor 160 selects, as the final bioreactor model, the candidate bioreactor model having the best simulation performance, based on the validation result. In other words, the processor 160 completes the bioreactor model using the parameter set having the smallest validation error among the estimated parameter sets.

The processor 160 determines the optimal operation condition of the bioreactor using the bioreactor model constructed via the bioreactor model construction process shown in FIG. 4, that is, using the final bioreactor model S30. In other words, the processor 160 determines the optimal feed rate of the bioreactor using the constructed bioreactor model.

Referring to FIG. 5 in order to describe the process of determining the optimal operation condition in more detail, the processor 160 selects the substrate feed rate change frequency K and the objective function J according to the user input S301. The substrate feed rate change frequency K means the number of times to change the substrate feed rate during the entire operation time duration of the bioreactor, and may be arbitrarily specified by the user. The objective function J may be set to the yield or the productivity. In other words, the processor 160 sets the substrate feed rate change frequency K and the objective function J according to the user input. In this embodiment, a case where the objective function J is set to the yield will be described by way of example.

When the substrate feed rate change frequency K and the objective function J are selected, the processor 160 calculates the substrate feed start time $t_{fs}$ S302. The processor 160 calculates, as the substrate feed start time $t_{fs}$, a time-point at which the substrate has been completely consumed in a first time-element in which the substrate is not fed to the bioreactor.

The processor 160 divides a time duration from the substrate feed start time tfs to the operation end time-point $t_{final}$ of the bioreactor into K time-elements, and calculates a time-point $t_k$ corresponding to a start time point of each of the K time-elements S303. That is, $t_k$ refers to the start time-point of each time-element, and $t_{k+1}$ means an end time-point of each time-element.

The processor 160 inputs the substrate feed rate in a $[t_k, t_{k+1}]$ time-element to the constructed bioreactor model to calculate the ordinary differential equation (ODE) and then calculates the change in the state variable S304. That is, the processor 160 calculates the ordinary differential equation (ODE) expressing the bioreactor model using the substrate feed rate in the $[t_k, t_{k+1}]$ time-element as a parameter and then calculates the change in the state variable.

Subsequently, the processor 160 identifies whether the operation scheme of the bioreactor is the MBO scheme S305. That is, the processor 160 identifies whether the operation scheme of the constructed bioreactor model is the MBO scheme.

When the operation scheme of the bioreactor is the MBO scheme, the processor 160 identifies whether there is a time-point at which the reactor volume V is greater than or equal to the maximum volume $V_{max}$ in the $[t_k, t_{k+1}]$ time-element S306. The processor 160 identifies whether there is a time-point at which the reactor volume V reaches the maximum volume $V_{max}$ in the $[t_k, t_{k+1}]$ time-element. In this connection, the processor 160 counts the number of MBO occurrences, that is, the MBO occurrence frequencies.

The processor 160 calculates a time-point at which the reactor volume V reaches the maximum volume $V_{max}$ in the $[t_k, t_{k+1}]$ time-element, that is, a MBO time-point $t_{MBO,j}$ S307. In this connection, $t_{MBO,j}$ means a time-point at which a j-th MBO occurs. When the operation scheme of the bioreactor is set to MBO, a MBO time-point and the MBO occurrence frequencies are calculated to find a portion where discontinuous manipulation is applied in a continuous process.

The processor 160 sets the reactor volume V to $V-V_{MBO}$ in the calculated MBO time-point $t_{MBO,j}$, and stores the ordinary differential equation (ODE) calculation result in a time duration up to the MBO time-point $t_{MBO,j}$ in the $[t_k, t_{k+1}]$ time-element, that is, in a $[t_k, t_{MBO,j}]$ time-element S308. The processor 160 calculates the change in the state variable in the $[t_k, t_{MBO,j}]$ time-element.

The processor 160 calculates the ordinary differential equation (ODE) of a $[t_{MBO,j}, t_{k+1}]$ time-element using the constructed bioreactor model, and calculates the change in the state variable in the $[t_{MBO,j}, t_{k+1}]$ time-element using the calculated ordinary differential equation (ODE) S309. In other words, the processor 160 calculates the state variable value using the ordinary differential equation (ODE) of the $[t_{MBO,j}, t_{k+1}]$ time-element, and calculates the change in the state variable in the $[t_{MBO,j}, t_{k+1}]$ time-element using the calculated state variable value. Then, the processor 160 returns to S306 to identify whether the reactor volume is greater than or equal to the maximum volume.

The processor 160 stores the calculation result of the change in the calculated state variable when the reactor volume V is smaller than the maximum volume Vmax S310. The processor 160 may store the ordinary differential equation (ODE) calculation result of the state variable in the $[t_{MBO,j}, t_{k+1}]$ time-element or the $[t_k, t_{k+1}]$ time-element.

The processor 160 identifies whether the change in the state variable on each of all time-elements has been calculated S311. That is, the processor 160 identifies whether the calculation of the change in the state variable on each of the K time-elements is completed.

The processor 160 inputs the calculation result of the change in the state variable to the genetic algorithm GA as a variable to determine the substrate feed rate to minimize the selected objective function J S312. In other words, the processor 160 derives the optimal substrate feed rate of the bioreactor that may maximize the yield or the productivity based on the result of calculating the change in the state variable.

The processor 160 outputs the determined optimal operation condition to the output device 130 S40. For example, the processor 160 displays the determined optimal operation condition on a display screen.

As described above, in accordance with the present disclosure, the optimal operation condition of the bioreactor is derived by parameterizing the substrate feed rate in each time-element and applying the substrate feed rate as a variable to the genetic algorithm GA.

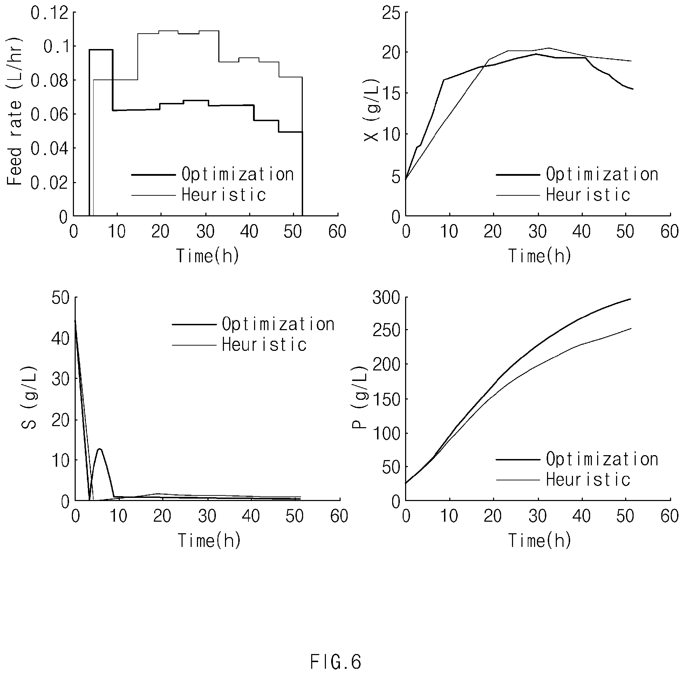

FIG. 6 is a graph showing change in a process indicator when operating a bioreactor according to the present disclosure.

Referring to FIG. 6, the optimal substrate feed rate predicted by the bioreactor model, that is, the optimal operation scheme is a scheme in which the feed rate is high at a beginning timing of the operation and a smaller total amount of the substrate is used, compared to a conventional operation scheme. Regarding the change in each process indicator when the bioreactor operates in the scheme according to the present disclosure, the cell concentration X increases rapidly at the beginning of the operation and then decreases due to a smaller feed at a later timing of the operation. Thus, the concentration S of the substrate is higher than that in the conventional operation scheme only at the beginning of the operation, thus helping increase the cell concentration. In a subsequent timing of the operation, there is no significant difference between the concentrations of the substrate in the scheme according to the present disclosure and the conventional operation scheme. Therefore, it may be identified that the product concentration P in the scheme according to the present disclosure increases, compared to the conventional operation scheme.

Hereinabove, although the present disclosure has been described with reference to exemplary embodiments and the accompanying drawings, the present disclosure is not limited thereto, but may be variously modified and altered by those skilled in the art to which the present disclosure pertains without departing from the spirit and scope of the present disclosure claimed in the following claims.

**Claims**

1. A method for determining an operating condition of a bioreactor, the method comprising:

   receiving N experimental data sets;
   modeling the bioreactor using the N experimental data sets to construct a bioreactor model; and
   determining an optimal operation condition based on an operation scheme of the bioreactor, using the constructed bioreactor model.

2. The method of claim 1, wherein the experimental data set includes a state variable and an operation condition of a previously-performed process.

3. The method of claim 2, wherein the state variable includes at least one of a cell concentration, a substrate concentration, a reactor volume, or a product concentration,
   wherein the operation condition includes at least one of a substrate feed rate, a temperature, pH (hydrogen exponent) or the operation scheme.

4. The method of claim 1, wherein the constructing of the bioreactor model includes:

   defining one experimental data set among the N experimental data sets as a validation data set, and defining remaining N-1 experimental data sets or some thereof as a training data set;
   estimating a parameter set using the training data set;
   creating candidate bioreactor models using the estimated parameter set;
   validating simulation performance of each of the created candidate bioreactor models, using the validation data set; and
   selecting a final bioreactor model among the created candidate bioreactor models, based on the simulation performance validation result.

5. The method of claim 4, wherein the validating of the simulation performance of each of the candidate bioreactor models includes:

   comparing a predicted value of each of the candidate bioreactor models with a measurement value of the validation data set; and
   calculating a validation error based on the comparing result.

6. The method of claim 5, wherein the selecting of the final bioreactor model includes selecting a candidate bioreactor model having a smallest validation error as the final bioreactor model, from among the candidate bioreactor models.

7. The method of claim 4, wherein the validating of the simulation performance of each of the candidate bioreactor models includes iteratively performing the validating of the simulation performance of each of the candidate bioreactor models while changing the training data set and the validation data set constituting the N experimental data sets.

8. The method of claim 1, wherein the determining of the optimal operation condition includes:

   dividing an entire operation time duration of the bioreactor into a plurality of time-elements;
   calculating change in a state variable based on each time-element-based substrate feed rate, using the constructed bioreactor model; and
   determining an optimal substrate feed rate that minimizes an objective function according to the operation scheme of the bioreactor, based on a calculation result of the change in the state variable.

9. The method of claim 8, wherein the dividing of the entire operation time duration of the bioreactor into the plurality of time-elements includes:

   selecting a substrate feed rate change frequency;
   calculating a substrate feed start time;

dividing a time duration from the substrate feed start time to an end time of an operation of the bioreactor into the plurality of time-elements, based on the substrate feed rate change frequency; and

calculating a start time point of each of the time-elements.

10. The method of claim 8, wherein the objective function is set to one of a yield and a productivity.

11. The method of claim 8, wherein the operation scheme includes a CBO (Continuous Broth Output) scheme or a MBO (Middle Broth Output) scheme.

12. The method of claim 11, wherein the determining of the optimal feed rate includes:

calculating a time-point at which a reactor volume reaches a maximum volume when the operation scheme is the MBO scheme;

setting the reactor volume at the calculated time-point to a value obtained by subtracting a MBO volume from the reactor volume, wherein the MBO volume is a product volume discharged from the bioreactor;

storing a result of calculating change in the state variable for a duration from a start time-point of a time-element in which the reactor volume reaches the maximum volume to the calculated time-point; and

calculating change in the state variable for a duration from the calculated time-point to an end time-point of the time-element in which the reactor volume reached the maximum volume, using the constructed bioreactor model, and storing the change.

13. A device for determining an operation condition of a bioreactor, the device comprising:

a memory; and

a processor connected to the memory,

wherein the processor is configured to:

receive N experimental data sets;

model the bioreactor using the N experimental data sets to construct a bioreactor model; and

determine an optimal operation condition based on an operation scheme of the bioreactor, using the constructed bioreactor model.

14. The device of claim 13, wherein the experimental data set includes a state variable and an operation condition of a previously-performed process,

wherein the state variable includes at least one of a cell concentration, a substrate concentration, a reactor volume, or a product concentration, and

wherein the operation condition includes at least one of a substrate feed rate, a temperature, pH (hydrogen exponent) or the operation scheme.

15. The device of claim 13, wherein the processor is configured to:

define one experimental data set among the N experimental data sets as a validation data set, and define remaining N-1 experimental data sets or some thereof as a training data set;

estimate a parameter set using the training data set;

create candidate bioreactor models using the estimated parameter set;

validate simulation performance of each of the created candidate bioreactor models, using the validation data set; and

select a final bioreactor model among the created candidate bioreactor models, based on the simulation performance validation result.

16. The device of claim 15, wherein the processor is configured to:

compare a predicted value of each of the candidate bioreactor models with a measurement value of the validation data set; and

calculate a validation error based on the comparing result.

17. The device of claim 16, wherein the processor is configured to select a candidate bioreactor model having a smallest validation error as the final bioreactor model, from among the candidate bioreactor models.

**18.** The device of claim 15, wherein the processor is configured to iteratively perform the validating of the simulation performance of each of the candidate bioreactor models while changing the training data set and the validation data set constituting the N experimental data sets.

**19.** The device of claim 15, wherein the processor is configured to:

divide an entire operation time duration of the bioreactor into a plurality of time-elements;
calculate change in a state variable based on each time-element-based substrate feed rate, using the constructed bioreactor model; and
determine an optimal substrate feed rate that minimizes an objective function according to the operation scheme of the bioreactor, based on a calculation result of the change in the state variable.

**20.** The device of claim 19, wherein the processor is configured to:

select a substrate feed rate change frequency;
calculate a substrate feed start time;
divide a time duration from the substrate feed start time to an end time of an operation of the bioreactor into the plurality of time-elements, based on the substrate feed rate change frequency; and
calculate a start time point of each of the time-elements.

**21.** The device of claim 15, wherein the processor is configured to:

calculate a time-point at which a reactor volume reaches a maximum volume when the operation scheme is a MBO scheme;
set the reactor volume at the calculated time-point to a value obtained by subtracting a MBO volume from the reactor volume, wherein the MBO volume is a product volume discharged from the bioreactor;
store a result of calculating change in the state variable for a duration from a start time-point of a time-element in which the reactor volume reaches the maximum volume to the calculated time-point; and
calculate change in the state variable for a duration from the calculated time-point to an end time-point of the time-element in which the reactor volume reached the maximum volume, using the constructed bioreactor model, and store the change.

160

PROCESSOR

110

MEMORY

120

INPUT
DEVICE

130

OUTPUT
DEVICE

140

STORAGE

150

NETWORK
INTERFACE

FIG.1

FIG.2

```
                        ┌───────────┐
                        │   START   │
                        └─────┬─────┘
                              │
                              ▼
        ┌─────────────────────────────────────────┐
        │     RECEIVE N EXPERIMENTAL DATA SETS     │──── S10
        └─────────────────────┬───────────────────┘
                              │
                              ▼
        ┌─────────────────────────────────────────┐
        │      CONSTRUCT BIOREACTOR MODEL USING    │──── S20
        │           EXPERIMENTAL DATA SETS         │
        └─────────────────────┬───────────────────┘
                              │
                              ▼
        ┌─────────────────────────────────────────┐
        │  DETERMINE OPTIMAL OPERATION CONDITION OF│──── S30
        │ BIOREACTOR USING CONSTRUCTED BIOREACTOR MODEL│
        └─────────────────────┬───────────────────┘
                              │
                              ▼
        ┌─────────────────────────────────────────┐
        │    OUTPUT DETERMINED OPTIMAL OPERATION   │──── S40
        │                CONDITION                 │
        └─────────────────────┬───────────────────┘
                              │
                              ▼
                        ┌───────────┐
                        │    END    │
                        └───────────┘
```

FIG.3

FIG.4

S20

SELECT SUBSTRATE FEED RATE CHANGE
FREQUENCY K AND OBJECTIVE FUNCTION J ～S301

CALCULATE SUBSTRATE FEED START
TIME $T_{FS}$ ～S302

DIVIDE TIME DURATION FROM SUBSTRATE
FEED START TIME $T_{FS}$ TO OPERATION END
TIME-POINT $T_{FINAL}$ OF BIOREACTOR INTO
K TIME-ELEMENTS, AND CALCULATE TIME
-POINT $T_K$ CORRESPONDING TO START TIME
POINT OF EACH OF K TIME-ELEMENTS ～S303

CALCULATE CHANGE IN STATE VARIABLE IN
$[T_T, T_{T+1}]$ TIME-ELEMENT USING FINAL
BIOREACTOR MODEL ～S304

WHETHER OPERATION
SCHEME OF BIOREACTOR IS
MBO SCHEME? S305
NO

YES

WHETHER THERE IS
TIME-POINT AT WHICH REACTOR
VOLUME IS GREATER THAN OR EQUAL
TO MAXIMUM VOLUME? S306
NO

YES

CALCULATE TIME-POINT AT WHICH REACTOR
VOLUME REACHES MAXIMUM VOLUME IN
$[T_K, T_{K+1}]$ TIME-ELEMENT, THAT IS,
MBO TIME-POINT $T_{MBO,J}$ ～S307

SET REACTOR VOLUME V TO V-$V_{MBO}$ IN
CALCULATED MBO TIME-POINT $T_{MBO,j}$, AND
CALCULATE AND STORE CHANGE IN STATE
VARIABLE IN $[T_K, T_{MBO,J}]$ TIME-ELEMENT ～S308

CALCULATE CHANGE IN STATE VARIABLE
IN $[T_{MBO,j}, T_{k+1}]$ TIME-ELEMENT USING
FINAL BIOREACTOR MODEL ～S309

STORE CALCULATION RESULT OF CHANGE
IN STATE VARIABLE IN $[T_{MBO,J}, T_{K+1}]$
TIME-ELEMENT OR $[T_K, T_{K+1}]$ TIME
-ELEMENT USING FINAL BIOREACTOR MODEL S310

WHETHER CHANGE
IN STATE VARIABLE ON EACH OF
ALL TIME-ELEMENTS HAS BEEN
CALCULATED? S311
NO

YES S312

DETERMINE SUBSTRATE FEED RATE TO
MINIMIZE SELECTED OBJECTIVE FUNCTION J

S40

FIG.5

FIG.6

## INTERNATIONAL SEARCH REPORT

| | International application No. |
|---|---|
| | **PCT/KR2020/000656** |

| A. | CLASSIFICATION OF SUBJECT MATTER |
|---|---|

*C12M 1/36(2006.01)i, C12M 1/34(2006.01)i, G05B 13/04(2006.01)i*

According to International Patent Classification (IPC) or to both national classification and IPC

| B. | FIELDS SEARCHED |
|---|---|

Minimum documentation searched (classification system followed by classification symbols)

C12M 1/36; A01G 7/00; A01H 13/00; B01J 19/00; C02F 3/30; C02F 9/14; C12M 1/00; C12N 1/00; G06G 7/58; C12M 1/34; G05B 13/04

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: bioreactor, operating condition

| C. | DOCUMENTS CONSIDERED TO BE RELEVANT | |
|---|---|---|
| Category* | Citation of document, with indication, where appropriate, of the relevant passages | Relevant to claim No. |
| X | WO 2009-148962 A1 (TUFTS UNIVERSITY) 10 December 2009<br>See abstract, page 6, claims 1-19. | 1-7,13-18 |
| A | | 8-12,19-21 |
| A | WO 2006-066002 A2 (BIOPROCESSORS CORP. et al.) 22 June 2006<br>See abstract; claims 1-14. | 1-21 |
| A | WO 2010-002745 A1 (SOLIX BIOFUELS, INC. et al.) 07 January 2010<br>See abstract; claims 1-55. | 1-21 |
| A | KR 10-2008-0063489 A (SIEMENS WATER TECHNOLOGIES CORP.) 04 July 2008<br>See abstract; claims 1-6. | 1-21 |
| A | JP 2008-526203 A (BIOGEN IDEC MA INC.) 24 July 2008<br>See the entire document. | 1-21 |

☐ Further documents are listed in the continuation of Box C.    ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 01 MAY 2020 (01.05.2020) | **01 MAY 2020 (01.05.2020)** |

| Name and mailing address of the ISA/KR | Authorized officer |
|---|---|
| Korean Intellectual Property Office<br>Government Complex Daejeon Building 4, 189, Cheongsa-ro, Seo-gu,<br>Daejeon, 35208, Republic of Korea | |
| Facsimile No. +82-42-481-8578 | Telephone No. |

Form PCT/ISA/210 (second sheet) (January 2015)

**INTERNATIONAL SEARCH REPORT**
Information on patent family members

International application No.

**PCT/KR2020/000656**

| Patent document cited in search report | Publication date | Patent family member | Publication date |
|---|---|---|---|
| WO 2009-148962 A1 | 10/12/2009 | None | |
| WO 2006-066002 A2 | 22/06/2006 | WO 2006-066002 A3 | 31/08/2006 |
| WO 2010-002745 A1 | 07/01/2010 | AU 2009-267214 A1 | 07/01/2010 |
| | | BR PI0914593 A2 | 15/12/2015 |
| | | CN 102131383 A | 20/07/2011 |
| | | CN 102131383 B | 23/07/2014 |
| | | MX 2010014547 A | 26/04/2011 |
| | | US 2012-0107921 A1 | 03/05/2012 |
| KR 10-2008-0063489 A | 04/07/2008 | AU 2006-299746 A1 | 12/04/2007 |
| | | AU 2006-299746 B2 | 04/08/2011 |
| | | CA 2622930 A1 | 12/04/2007 |
| | | CA 2622930 C | 19/01/2016 |
| | | CN 101277905 A | 01/10/2008 |
| | | CN 101277905 B | 07/08/2013 |
| | | EP 1931603 A1 | 18/06/2008 |
| | | EP 1931603 B1 | 10/12/2014 |
| | | ES 2530790 T3 | 05/03/2015 |
| | | HU E024179 T4 | 30/05/2016 |
| | | JP 2009-509756 A | 12/03/2009 |
| | | NZ 566779 A | 31/03/2011 |
| | | US 2008-0314829 A1 | 25/12/2008 |
| | | US 7655142 B2 | 02/02/2010 |
| | | WO 2007-0038843 A1 | 12/04/2007 |
| JP 2008-526203 A | 24/07/2008 | AU 2005-322159 A1 | 06/07/2006 |
| | | CA 2593374 A1 | 06/07/2006 |
| | | CN 101443444 A | 27/05/2009 |
| | | EP 1844138 A2 | 17/10/2007 |
| | | US 2009-0104594 A1 | 23/04/2009 |
| | | WO 2006-071716 A2 | 06/07/2006 |
| | | WO 2006-071716 A3 | 05/03/2009 |

Form PCT/ISA/210 (patent family annex) (January 2015)

**EP 3 926 035 A1**

**Patent documents cited in the description**

- KR 100924065

- KR 1208480